(19) Europäisches Patentamt

European Patent Office

Office européen des brevets

(11) **EP 3 255 091 B1**

(12) **EUROPEAN PATENT SPECIFICATION**

(45) Date of publication and mention
of the grant of the patent:
**19.06.2019 Bulletin 2019/25**

(21) Application number: **17165173.0**

(22) Date of filing: **06.04.2017**

(51) Int Cl.:
**C08L 21/00** *(2006.01)*   **C08L 9/06** *(2006.01)*
**C08C 19/25** *(2006.01)*   **G01N 23/201** *(2018.01)*
**G01N 23/202** *(2006.01)*

(54) **RUBBER COMPOSITION, PRODUCTION METHOD THEREFOR, AND METHOD FOR EVALUATING WEAR RESISTANCE OF RUBBER COMPOSITION**

KAUTSCHUKZUSAMMENSETZUNG, HERSTELLUNGSVERFAHREN DAFÜR UND VERFAHREN ZUR BEWERTUNG DER VERSCHLEISSBESTÄNDIGKEIT DER KAUTSCHUKZUSAMMENSETZUNG

COMPOSITION DE CAOUTCHOUC, PROCÉDÉ DE PRODUCTION ASSOCIÉ ET PROCÉDÉ D'ÉVALUATION DE LA RÉSISTANCE À L'USURE D'UNE COMPOSITION DE CAOUTCHOUC

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.06.2016 JP 2016115438**

(43) Date of publication of application:
**13.12.2017 Bulletin 2017/50**

(73) Proprietor: **Sumitomo Rubber Industries, Ltd.
Kobe-shi,
Hyogo-ken 651-0072 (JP)**

(72) Inventors:
• **MASHITA, Ryo
Kobe-shi, Hyogo 651-0072 (JP)**
• **KISHIMOTO, Hiroyuki
Kobe-shi, Hyogo 651-0072 (JP)**
• **KITAGO, Ryota
Kobe-shi, Hyogo 651-0072 (JP)**

(74) Representative: **Manitz Finsterwald
Patent- und Rechtsanwaltspartnerschaft mbB
Martin-Greif-Strasse 1
80336 München (DE)**

(56) References cited:
**EP-A1- 1 958 971    EP-A1- 2 098 554
EP-A1- 2 735 865    EP-A1- 2 749 874
EP-A1- 2 799 482    EP-A1- 2 803 982
EP-A2- 2 557 410**

**Description**

BACKGROUND OF THE INVENTION

Field of the Invention

The present invention relates to a rubber composition, a production method therefor, and a method for evaluating the wear resistance of a rubber composition.

Description of the Background Art

A rubber composition used for a tire tread portion generally tends to have better wear resistance as the molecular weight of a rubber component used therein increases (see, for example, Japanese Laid-Open Patent Publication No. 2013-32497). One factor of this is thought to be that the number of polymer terminals that can be start points of breakage reduces as the molecular weight increases.

However, the wear resistance does not necessarily improve when the molecular weight increases, and there are some exceptions. Hitherto, the wear phenomenon of rubber has been observed by various methods in order to unravel the mechanism for creation of such exceptions, but the mechanism has not fully been unraveled. Further prior art methods are disclosed in the following documents EP2735865, EP2749874, EP2803982, EP1958971, EP2098554, EP2557410 and EP2799482.

SUMMARY OF THE INVENTION

The present invention has been made in order to solve the above problem, and an object of the present invention is to provide a rubber composition having excellent wear resistance, a production method therefor, and a method by which the wear resistance of a rubber composition can be evaluated with high accuracy.

As a result of examination, the present inventors have found that a standard deviation $\sigma_c$ of a maximum value $\Xi_c$ of a correlation length $\Xi_i$ of 10 nm to 100 $\mu$m obtained by performing curve-fitting with specific expressions on a scattering intensity curve obtained by performing X-ray scattering measurement or neutron scattering measurement in a specific region exhibits a high correlation with the wear resistance of a rubber composition, and the wear resistance tends to become more favorable as the standard deviation $\sigma_c$ decreases. This is inferred to be because an ununiform network structure, in a polymer, represented by the correlation length $\Xi_i$ serves to inhibit crack growth, and as the size of the network structure is more uniform, the number of locations on which stress is concentrated is reduced, and the crack growth inhibition effect is enhanced, so that the wear resistance improves.

As a result of further examination, the present inventors have found that particularly favorable wear resistance is achieved with a rubber composition which contains a rubber component having a weight average molecular weight of not less than one million and in which a maximum value $\Xi_c$ and a standard deviation $\sigma_c$ satisfy a relationship of $\sigma_c \leq \Xi_c/2$, and have arrived at the present invention. That is, the present invention is directed to a rubber composition in which a relationship between: a maximum value $\Xi_c$ of a correlation length $\Xi_i$ of 10 nm to 100 $\mu$m obtained by performing curve-fitting with the following (Expression 1-2) to (Expression 1-7) on a scattering intensity curve I(q) obtained by performing X-ray scattering measurement or neutron scattering measurement in a region of q represented by the following (Expression 1); and a standard deviation $\sigma_c$ of the maximum value $\Xi_c$ is $\sigma_c \leq \Xi_c/2$, the rubber composition containing a rubber component having a weight average molecular weight of not less than one million, and wherein an amount of carbon black with respect to 100 parts by mass of the rubber component is not less than 1 part by mass and is not greater than 20 parts by mass.

[Math. 1]

$$(\text{Expression 1})$$

$$q = \frac{4\pi \sin(\theta/2)}{\lambda}$$

($\theta$: scattering angle, $\lambda$: a wavelength of X-rays or neutron rays)

[Math. 2]

(Expression 1-2)

$$I_{(q)} = \int \frac{A}{1 + q^2\xi^2} g_{a(r)} dr + \sum_{i=1}^{n} \int \frac{B_i}{(1 + q^2\Xi_i^2)^2} g_{bi(r)} dr$$

(Expression 1-3)

$$\xi < \Xi_i$$

(Expression 1-4)

$$A = 8\pi N_a \rho^2 \xi^3$$

(Expression 1-5)

$$B_i = 4\pi N_i \rho^2 \Xi_i^2$$

(Expression 1-6)

$$g_{a(r)} = \frac{1}{\sqrt{2\pi\sigma_a^2}} exp\left(-\frac{(r-\xi)^2}{2\sigma_a^2}\right)$$

(Expression 1-7)

$$g_{bi(r)} = \frac{1}{\sqrt{2\pi\sigma_i^2}} exp\left(-\frac{(r-\Xi_i)^2}{2\sigma_i^2}\right)$$

(A, $B_i$, $\xi$, $\Xi_i$: fitting parameters)
(n: an integer)
(q: the same as in (Expression 1))
($N_a$: the number of scatterers having a correlation length $\xi$ per unit volume (number/cm$^3$))
($N_i$: the number of scatterers having the correlation length $\Xi_i$ per unit volume (number/cm$^3$))
(p: a scattering length density difference (cm$^{-2}$) or an electron density difference (electron cm$^{-3}$) between the scatterers and a deuterated solvent or air therearound)
(ga(r): a probability density function having an average correlation length $\xi$ and a standard deviation $\sigma_a$ when a correlation length is assumed to be present in a Gaussian distribution)
(gbi(r): a probability density function having an average correlation length $\Xi_i$ and a standard deviation $\sigma_i$ when a correlation length is assumed to be present in a Gaussian distribution)

The rubber component is preferably a polymer of one or more conjugated diene compounds.

The rubber composition is preferably used for a tire member.

Preferably, the X-ray scattering measurement is small-angle X-ray scattering measurement, and the neutron scattering measurement is small-angle neutron scattering measurement.

The X-ray scattering measurement or the neutron scattering measurement is preferably performed in a region of q that is not greater than 10 nm$^{-1}$.

The present invention is also directed to a production method for the rubber composition according to any one of claims 1 to 5, the production method comprising the step of kneading the rubber component, a sulfur donor, and a sulfur atom-containing vulcanization accelerator before the rubber component and a filler are kneaded, then adding the filler to an obtained kneaded product, and performing kneading at a kneading temperature of not lower than 120°C.

The present invention is also directed to a method for evaluating wear resistance of a rubber composition by X-ray scattering measurement or neutron scattering measurement, the method comprising evaluating the wear resistance of the rubber composition on the basis of a standard deviation $\sigma_c$ of a maximum value $\Xi_c$ of a correlation length $\Xi_i$ of 10 nm to 100 $\mu$m obtained by performing curve-fitting with the above (Expression 1-2) to (Expression 1-7) on a scattering intensity curve I(q) obtained by performing the X-ray scattering measurement or the neutron scattering measurement in a region of q represented by the above (Expression 1).

According to the present invention, excellent wear resistance is achieved because of the rubber composition in which the relationship between: the maximum value $\Xi_c$ of the correlation length $\Xi_i$ of 10 nm to 100 $\mu$m obtained by performing curve-fitting with the above (Expression 1-2) to (Expression 1-7) on the scattering intensity curve I(q) obtained by performing X-ray scattering measurement or neutron scattering measurement in a region of q represented by the above (Expression 1); and the standard deviation $\sigma_c$ of the maximum value $\Xi_i$ is $\sigma_c \leq \Xi_c/2$, the rubber composition containing a rubber component having a weight average molecular weight of not less than one million.

In addition, it is possible to evaluate the wear resistance of a rubber composition with high accuracy by using $\sigma_c$ as an index for the wear resistance.

BRIEF DESCRIPTION OF THE DRAWINGS

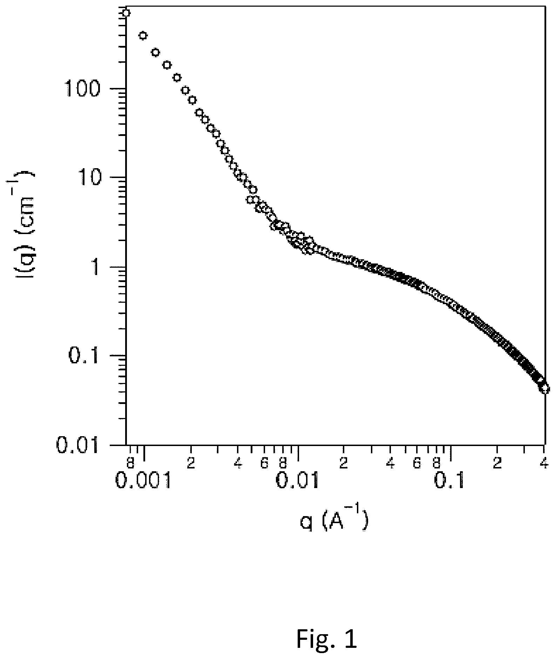

FIG. 1 shows an example of a scattering intensity curve obtained by SANS measurement.

DESCRIPTION OF THE PREFERRED EMBODIMENTS

The present invention is a rubber composition in which a relationship between: a maximum value $\Xi_c$ of a correlation length $\Xi_i$ of 10 nm to 100 $\mu$m obtained by performing curve-fitting with the above (Expression 1-2) to (Expression 1-7) on a scattering intensity curve I(q) obtained by performing X-ray scattering measurement or neutron scattering measurement in a region of q represented by the above (Expression 1); and a standard deviation $\sigma_c$ of the maximum value $\Xi_c$ is $\sigma_c \leq \Xi_c/2$, the rubber composition containing a rubber component having a weight average molecular weight of not less than one million.

As the X-ray scattering measurement, small-angle X-ray scattering (SAXS (scattering angle: normally 10 degrees or less)) measurement in which a rubber composition is irradiated with X-rays and a scattering intensity is measured, can suitably be adopted. In the small-angle X-ray scattering, of X-rays that are applied to a substance and scattered, the X-rays having a small scattering angle are measured, whereby structure information of the substance is obtained, so that an ordered structure such as the microphase-separated structure of a polymeric material can be analyzed at a level of several nanometers.

In order to obtain detailed molecular structure information from the SAXS measurement, it is desirable that an X-ray scattering profile with a high S/N ratio be measurable. Thus, X-rays emitted from a synchrotron preferably have a brightness of not less than at least $10^{10}$ (photons/s/mrad$^2$/mm$^2$/0.1% bw). The "bw" represents the band width of the X-rays emitted from the synchrotron. Examples of such a synchrotron include the beamlines BL03XU and BL20XU at the large-scale synchrotron radiation facility SPring-8 of the Japan Synchrotron Radiation Research Institute, which is an incorporated foundation.

The brightness (photons/s/mrad$^2$/mm$^2$/0.1% bw) of the X-rays is preferably not less than $10^{10}$ and more preferably not less than $10^{12}$. The upper limit thereof is not particularly limited, but the intensity of the X-rays is preferably not greater than the intensity that does not cause radiation damage.

Also, the number of photons (photons/s) in the X-rays is preferably not less than $10^7$ and more preferably not less than $10^9$. The upper limit thereof is not particularly limited, but the intensity of the X-rays is preferably not greater than the intensity that does not cause radiation damage.

As the neutron scattering measurement, small-angle neutron scattering (SANS (scattering angle: normally 10 degrees or less)) measurement in which a rubber composition is irradiated with neutron rays to measure the scattering intensity, can be suitably adopted. In the small-angle neutron scattering, of neutron rays that are applied to a substance and scattered, the neutron rays having a small scattering angle are measured, structure information of the substance is obtained, so that an ordered structure such as the microphase-separated structure of a polymeric material can be analyzed at a level of several nanometers.

In the SANS measurement, known methods that use magnetic structures or deuteration techniques can be used. In the case where a deuteration technique is used, for example, a polymeric material is swollen in a deuterated solvent, and the polymeric material in equilibrium in the deuterated solvent is then irradiated with neutron rays to measure the scattering intensity. Here, examples of deuterated solvents used to swell the polymeric material include heavy water, deuterated hexane, deuterated toluene, deuterated chloroform, deuterated methanol, deuterated DMSO ($(D_3C)_2S=O$), deuterated tetrahydrofuran, deuterated acetonitrile, deuterated dichloromethane, deuterated benzene, and deuterated N,N-dimethylformamide.

The neutron rays used for neutron scattering measurement such as SANS may be obtained by using, for example, the beamline SANS-J at JRR-3 of the Japan Atomic Energy Agency, which is an independent administrative agency, etc.

Similarly to the SAXS measurement, from the standpoint that a neutron scattering profile with a high S/N ratio is obtained, the neutron flux intensity of the neutron rays (neutrons/cm$^2$/s) is preferably not less than $10^3$ and more preferably not less than $10^4$. The upper limit thereof is not particularly limited, but the neutron flux intensity is preferably not greater than the neutron flux intensity that does not cause radiation damage.

The X-ray or neutron scattering measurement is performed in a region of q represented by the following (Expression 1). Regarding a region of q (nm$^{-1}$), information about a more detailed molecular structure is obtained as the numeric value increases. Thus, the X-ray or neutron scattering measurement is performed in a region of q that is not greater than 10 nm$^{-1}$ and more preferably in a region of q that is not greater than 20 nm$^{-1}$.

[Math. 3]

(Expression 1)

$$q = \frac{4\pi \sin(\theta/2)}{\lambda}$$

($\theta$: scattering angle, $\lambda$: a wavelength of X-rays or neutron rays)

The scattered X-rays in the SAXS measurement are detected by an X-ray detector, and an image is then generated by an image processor or the like using X-ray detection data from the X-ray detector.

As the X-ray detectors, for example, a two-dimensional detectors (e.g., an X-ray film, a nuclear emulsion plate, an X-ray image pickup tube, an X-ray fluorescent amplifier tube, an X-ray image intensifier, an X-ray imaging plate, a CCD for X-rays, an amorphous body for X-rays, etc.) or a one-dimensional detector (a line sensor) can be used. The X-ray detector may be appropriately selected according to the type and the state of a polymeric material to be analyzed, and the like.

Any ordinary image processor that can generate an X-ray scattering image on the basis of X-ray detection data from the X-ray detector can be appropriately used.

The SANS measurement can also be performed on the basis of the same principle as in the SAXS measurement. The scattered neutron rays are detected by a neutron ray detector, and an image is then generated by an image processor or the like using neutron ray detection data from the neutron ray detector. Here, similarly to the above, any known two-dimensional detector or one-dimensional detector can be used as the neutron ray detector, and any known image processor that can generate a neutron ray scattering image can be used as the image processor. These devices may be appropriately selected.

Then, for the rubber composition, a correlation length $\Xi_i$ of 10 nm to 100 $\mu$m, a maximum value $\Xi_c$ thereof, and a standard deviation $\sigma_c$ of the maximum value $\Xi_c$ can be calculated by performing measurement such as the SAXS measurement or the SANS measurement and analyzing the obtained scattering intensity curve by the following method.

First, curve-fitting is performed with the following (Expression 1-2) to (Expression 1-7) on a scattering intensity curve I(q) obtained by the SAXS measurement or the SANS measurement as shown in FIG. 1, and fitting parameters are obtained by a least-squares method.

[Math. 4]

(Expression 1-2)

$$I_{(q)} = \int \frac{A}{1 + q^2 \xi^2} g_{a(r)} dr + \sum_{i=1}^{n} \int \frac{B_i}{(1 + q^2 \Xi_i^2)^2} g_{bi(r)} dr$$

(Expression 1-3)

$$\xi < \Xi_i$$

(Expression 1-4)

$$A = 8\pi N_a \rho^2 \xi^3$$

(Expression 1-5)

$$B_i = 4\pi N_i \rho^2 \Xi_i^2$$

(Expression 1-6)

$$g_{a(r)} = \frac{1}{\sqrt{2\pi\sigma_a^2}} exp\left(-\frac{(r-\xi)^2}{2\sigma_a^2}\right)$$

(Expression 1-7)

$$g_{bi(r)} = \frac{1}{\sqrt{2\pi\sigma_i^2}} exp\left(-\frac{(r-\Xi_i)^2}{2\sigma_i^2}\right)$$

(A, $B_i$, $\xi$, $\Xi_i$: fitting parameters)
(n: an integer)
(q: the same as in (Expression 1))
($N_a$: the number of scatterers having a correlation length $\xi$ per unit volume (number/cm$^3$))
($N_i$: the number of scatterers having the correlation length $\Xi_i$ per unit volume (number/cm$^3$))
(p: a scattering length density difference (cm$^{-2}$) or an electron density difference (electron cm$^{-3}$) between the scatterers and a deuterated solvent or air therearound)
(ga(r): a probability density function having an average correlation length $\xi$ and a standard deviation $\sigma_a$ when a correlation length is assumed to be present in a Gaussian distribution)
(gbi(r): a probability density function having an average correlation length $\Xi_i$ and a standard deviation $\sigma_i$ when a correlation length is assumed to be present in a Gaussian distribution)

Among the obtained fitting parameters, the correlation length $\xi$ is presumed to correspond to the distance between crosslinking points in the polymer, and the correlation length $\Xi_i$ is presumed to correspond to the size of an ununiform network structure in the polymer. As described above, the standard deviation $\sigma_c$ of the maximum value $\Xi_c$ of the correlation length $\Xi_i$ has a high correlation with wear resistance, and the wear resistance becomes more favorable as $\sigma_c$ decreases. Thus, it is possible to evaluate the wear resistance with high accuracy by using $\sigma_c$ as an index for the wear resistance.

Regarding the above (Expression 1-7) representing the distribution of the correlation length $\Xi_i$, the Gaussian function is used on the assumption that $\Xi_i$ has a normal distribution. However, a gamma function, a Weibull function, or the like may be used as another distribution function.

A rubber composition having excellent wear resistance is obtained by performing adjustment so as to satisfy a relationship of $\sigma_c \leq \Xi_c/2$. For the reason that more favorable wear resistance is achieved, $\sigma_c$ and $\Xi_c$ preferably satisfy a relationship of $\sigma_c \leq \Xi_c/3$ and more preferably satisfy a relationship of $\sigma_c \leq \Xi_c/4$. In addition, $\Xi_c$ and $\sigma_c$ only need to satisfy the above relationship, but for the reason that more favorable wear resistance is achieved, $\Xi_c$ is preferably 500 to 1800 nm and more preferably 600 to 1500 nm, and $\sigma_c$ is preferably 50 to 500 nm and more preferably 100 to 300 nm.

$\Xi_i$, $\Xi_c$, and $\sigma_c$ can be adjusted on the basis of the types and the blending amounts of chemicals such as the rubber component, a filler, and the like to be blended in the rubber composition.

In addition, $\Xi_i$, $\Xi_c$, and $\sigma_c$ can also be adjusted on the basis of a kneading method. For example, a rubber composition that satisfies the relationship of $\sigma_c \leq \Xi_c/2$ can easily be prepared by a production method including a step of kneading the rubber component, a sulfur donor, and a sulfur atom-containing vulcanization accelerator before the rubber component and a filler are kneaded, then adding the filler to the obtained kneaded product, and performing kneading at a kneading temperature of not lower than 120°C.

By kneading the rubber component, the sulfur donor, and the sulfur atom-containing vulcanization accelerator before the rubber component and the filler are kneaded, absorption of the sulfur donor and the sulfur atom-containing vulcanization accelerator by the filler can be prevented, so that the sulfur donor and the sulfur atom-containing vulcanization accelerator can efficiently be dispersed in the rubber component. Then, by adding the filler to the kneaded product obtained by kneading the rubber component, the sulfur donor, and the sulfur atom-containing vulcanization accelerator and performing kneading at a kneading temperature of not lower than 120°C, active sulfur is released from the sulfur donor. The active sulfur, the sulfur atom-containing vulcanization accelerator, and the rubber component react with each other to obtain a state where the entirety or a part (hereinafter, "vulcanization accelerator residue") of the sulfur atom-containing vulcanization accelerator is bonded to the rubber component, that is, a state where a pendant type structure is formed in which "-S- vulcanization accelerator residue" is bonded to the rubber component. The mechanism of this reaction is inferred to be that the released active sulfur reacts with the sulfur atoms of the sulfur atom-containing vulcanization accelerator to form a structure in which two or more sulfur atoms are bonded, and the structure portion and the double-bond portion of the rubber component react with each other. By performing kneading in the state where the pendant-type structure is formed, the vulcanization accelerator residue moves together with the rubber component. Thus, the uniformity of the dispersion state of the vulcanization accelerator residue in the entire rubber composition improves, so that the crosslink density during vulcanization can be uniformized. The kneading temperature is the actual measured temperature of the kneaded product in a kneading machine and can be measured by a noncontact type temperature sensor or the like.

The production method is characterized in that: kneading of the rubber component, the sulfur donor, and the sulfur atom-containing vulcanization accelerator is started before the filler is kneaded; and kneading is performed at a kneading temperature of not lower than 120°C after the filler is added, as described above. As long as the above requirements are met, any of the materials may be added in any step. For example, in the case where the kneading step includes two steps composed of a step X and a step F, kneading of the rubber component, the sulfur donor, and the sulfur atom-containing vulcanization accelerator may be started at the initial stage of the step X, the filler may be added and kneading may be performed at a kneading temperature of not lower than 120°C in the middle of the step X, and then the step F may be performed. In addition, in the case where the kneading step includes three steps composed of a step X, a step Y, and a step F, kneading of the rubber component, the sulfur donor, and the sulfur atom-containing vulcanization accelerator may be started in the step X, the filler may be added and kneading may be performed at a kneading temperature of not lower than 120°C in the subsequent step Y, and then step F may be performed. Moreover, in another example of the case with three steps, kneading of the rubber component, the sulfur donor, and the sulfur atom-containing vulcanization accelerator may be started at the initial stage of the step X, the filler may be added and kneading may be performed at a kneading temperature of not lower than 120°C in the middle of the step X, and then step Y and the step F may be performed; or kneading of the rubber component, the sulfur donor, and the sulfur atom-containing vulcanization accelerator may be started at the initial stage of the step X, the filler may be added in the middle of the step X, the filler may be further added and kneading may be performed at a kneading temperature of not lower than 120°C in the subsequent step Y, and then the step F may be performed. Re-milling may be performed between the respective steps.

The kneading temperature at which the rubber component, the sulfur donor, and the sulfur atom-containing vulcanization accelerator are kneaded is not particularly limited. However, from the standpoint of inhibiting progress of a crosslinking reaction caused by the sulfur donor and the sulfur atom-containing vulcanization accelerator, the kneading temperature is preferably less than 160°C and more preferably not higher than 150°C. The lower limit thereof is not particularly limited, but the kneading temperature is preferably not lower than 60°C.

The kneading time for which the rubber component, the sulfur donor, and the sulfur atom-containing vulcanization accelerator are kneaded before the filler is added to the rubber component is not particularly limited. However, from the standpoint of improvement of the dispersibility of the sulfur donor and the sulfur atom-containing vulcanization accelerator, the kneading time is, for example, not shorter than 10 seconds. The upper limit thereof is not particularly limited, but the kneading time is preferably not longer than 8 minutes.

The kneading temperature after the filler is added only needs to be not lower than 120°C. However, from the standpoint of inhibiting excessive progress of a crosslinking reaction, the kneading temperature is preferably not lower than 170°C.

In addition, the kneading time after the filler is added to the rubber component and the kneading temperature reaches 120°C is not particularly limited. However, from the standpoint of improvement of the dispersibility of the sulfur donor and the sulfur atom-containing vulcanization accelerator, the kneading time is preferably not shorter than 2 minutes. The upper limit thereof is not particularly limited, but the kneading time is preferably not longer than 10 minutes. This kneading time is the time from the time point at which the filler is added to the rubber component and the kneading temperature reaches 120°C to the time point at which all the steps of the kneading step end. For example, when the filler is added to the rubber component and the kneading temperature reaches 120°C in the step X, this kneading time is the time from this time point to the time point at which the step F (final kneading step) ends.

In the present invention, a rubber component having a weight average molecular weight of not less than one million is used. Accordingly, the number of start points of breakage decreases, and favorable wear resistance is achieved. For the reason that more favorable wear resistance is achieved, the weight average molecular weight is preferably not less than 1.2 million and more preferably not less than 1.4 million. The upper limit thereof is not particularly limited, but the weight average molecular weight is preferably not greater than 2.5 million. In the present invention, the weight average molecular weight (Mw) is a value obtained by measuring the entire rubber component blended in the rubber composition by a method in Examples. In the case where a plurality of types are used in combination as the rubber component, the weight average molecular weight (Mw) is a value obtained by measuring a mixture of the plurality of types.

As the rubber component, a polymer in which one or more conjugated diene compounds such as isoprene and butadiene are used, can be used. More specifically, as the rubber component, natural rubber (NR), isoprene rubber (IR), butadiene rubber (BR), styrene butadiene rubber (SBR), acrylonitrile butadiene rubber (NBR), chloroprene rubber (CR), isobutylene-isoprene-rubber (IIR), halogenated isobutylene-isoprene-rubber (X-IIR), styreneisoprene-butadiene rubber (SIBR), or the like can be used. One of them may be used singly, or two or more of them may be used in combination. Among them, the SBR and the BR are preferable.

In addition, the SBR is preferably modified SBR in which the main chain thereof is modified by a nitrogen-containing compound, and at least one terminal thereof is modified by a terminal modifier. Examples of the nitrogen-containing compound include 3- or 4-(2-azetidinoethyl)styrene, 3- or 4-(2-pyrrolidinoethyl)styrene, 3- or 4-(2-piperidinoethyl)styrene, and 3- or 4-(2-hexamethyleneiminoethyl)styrene. Among them, 3- or 4-(2-pyrrolidinoethyl)styrene is preferable. Moreover, the terminal modifier is preferably a silicon-containing compound, and examples thereof include 3-(N,N-dimethylaminopropyl)trimethoxysilane, 3-(N,N-diethylaminopropyl)trimethoxysilane, 3-(N,N-dimethylaminopropyl)triethoxysilane, 3-(N,N-diethylaminopropyl)triethoxysilane, 3-glycidoxypropyltrimethoxysilane, 2-(4-pyridylethyl)triethoxysilane, N-(3-triethoxysilylpropyl)-4,5-dihydroimidazole, and silicon tetrachloride. Among them, 3-(N,N-dimethylaminopropyl)trimethoxysilane is preferable.

In the rubber composition of the present invention, the amount of the SBR in 100% by mass of the rubber component is preferably not less than 40% by mass and more preferably not less than 60% by mass, and is preferably not greater than 95% by mass and more preferably not greater than 85% by mass.

In the rubber composition of the present invention, the amount of the BR in 100% by mass of the rubber component is preferably not less than 5% by mass and more preferably not less than 15% by mass, and is preferably not greater than 60% by mass and more preferably not greater than 40% by mass.

As the filler, a reinforcing filler such as silica and carbon black which are generally used in the tire field can be used.

In the rubber composition of the present invention, the amount of silica with respect to 100 parts by mass of the rubber component is preferably not less than 30 parts by mass and more preferably not less than 60 parts by mass, and is preferably not greater than 120 parts by mass and more preferably not greater than 90 parts by mass.

In the rubber composition of the present invention, the amount of carbon black with respect to 100 parts by mass of the rubber component is not less than 1 part by mass and preferably not less than 3 parts by mass, and is not greater than 20 parts by mass and preferably not greater than 10 parts by mass.

The sulfur donor is elemental sulfur or a sulfur compound that releases active sulfur under a vulcanization condition (e.g., 150°C, 1.5 MPa) or at a temperature lower than 150°C and under a pressure lower than 1.5 MPa. In other words, the sulfur donor is a compound that generally functions as a vulcanizing agent under a vulcanization condition (e.g., 150°C, 1.5 MPa) or at a temperature lower than 150°C and under a pressure lower than 1.5 MPa. The released active sulfur forms a part of the above-described pendant type structure.

As the sulfur donor, elemental sulfur and/or the above-described sulfur compound that releases active sulfur can be used. Examples of the elemental sulfur include powdery sulfur, precipitated sulfur, colloidal sulfur, surface-treated sulfur, and insoluble sulfur.

When the elemental sulfur is blended in an excessive amount as the sulfur donor, a vulcanization reaction may excessively progress in the kneading step. Thus, in the rubber composition of the present invention, in the case where the elemental sulfur is used as the sulfur donor, the amount of the elemental sulfur to be added before the rubber component and the filler are kneaded, is preferably not greater than 0.1 parts by mass with respect to 100 parts by mass of the rubber component (the total amount of the rubber component to be used in all the steps). In addition, the amount is preferably not less than 0.05 parts by mass from the standpoint of breaking strength.

Examples of the sulfur compound that serves as the sulfur donor include a polymeric polysulfide represented by $-(-M-S-C-)_n-$, and a compound that has a structure $-S_n-$ ($n \geq 2$) in which two or more sulfur atoms are each bonded by single bond and that releases active sulfur. Examples of the compound include alkylphenol disulfide, morpholine disulfide, thiuram-based vulcanization accelerators having $-S_n-$ ($n \geq 2$) (tetramethylthiuram disulfide (TMTD), tetraethylthiuram disulfide (TETD), tetrabutylthiuram disulfide (TBTD), dipentamethylenethiuram tetrasulfide (DPTT), etc.), 2-(4'-morpholinodithio)benzothiazole (MDB), and polysulfide type silane coupling agents (e.g., Si69 manufactured by Evonik Industries Ag (bis(3-triethoxysilylpropyl)tetrasulfide)). One of them may be used singly, or two or more of them may be used in combination. Among them, thiuram-based vulcanization accelerators having $-S_n-$ ($n \geq 2$) are preferable, and dipentamethylenethiuram tetrasulfide (DPTT) is more preferable.

In the rubber composition of the present invention, in the case where the sulfur compound is used as the sulfur donor, the amount of the sulfur compound to be added before the rubber component and the filler are kneaded is preferably not less than 0.1 parts by mass and more preferably not less than 0.2 parts by mass, with respect to 100 parts by mass of the rubber component (the total amount of the rubber component to be used in all the steps), for the reason of accelerating formation of the pendant type structure. In addition, from the standpoint of inhibiting gelling during kneading, the amount is preferably not greater than 5 parts by mass, more preferably not greater than 3 parts by mass, and further preferably not greater than 2 parts by mass.

The sulfur atom-containing vulcanization accelerator refers to a vulcanization accelerator containing a sulfur atom that is bonded to another molecule by single bond. Examples of the sulfur atom-containing vulcanization accelerator include a sulfur atom-containing vulcanization accelerator that releases active sulfur, and a sulfur atom-containing vulcanization accelerator that does not release active sulfur. From the standpoint of inhibiting progress of a crosslinking reaction during kneading, a sulfur atom-containing vulcanization accelerator that does not release active sulfur (a sulfur non-releasing type sulfur atom-containing vulcanization accelerator) is preferable.

Among the sulfur atom-containing vulcanization accelerators, there are vulcanization accelerators that serve as a sulfur donor (e.g., a vulcanization accelerator containing a sulfur atom that is bonded to another molecule by single bond). Therefore, it is possible to form the pendant type structure also by blending one of the sulfur atom-containing vulcanization accelerators that serve as a sulfur donor, in a large amount, or by using two or more of the sulfur atom-containing vulcanization accelerators, in combination. However, when the sulfur atom-containing vulcanization accelerator that serves as a sulfur donor is blended in a large amount, a crosslinking reaction during kneading may excessively progress. When the sulfur atom-containing vulcanization accelerator that serves as a sulfur donor is blended in a small amount, it may be difficult to achieve the effect of uniformizing the crosslink density. Thus, the sulfur donor and the sulfur atom-containing vulcanization accelerator that are kneaded before the filler is added is preferably a combination of a sulfur donor (the sulfur atom-containing vulcanization accelerator that serves as a sulfur donor and/or a sulfur donor other than the sulfur atom-containing vulcanization accelerator) and the sulfur non-releasing type sulfur atom-containing vulcanization accelerator (a sulfur atom-containing vulcanization accelerator that does not serve as a sulfur donor).

The sulfur non-releasing type sulfur atom-containing vulcanization accelerator refers to, for example, a sulfur atom-containing vulcanization accelerator that does not release active sulfur under a vulcanization condition (e.g., 150°C, 1.5 MPa) or at a temperature lower than 150°C and under a pressure lower than 1.5 MPa. In other words, the sulfur non-releasing type sulfur atom-containing vulcanization accelerator is a sulfur atom-containing vulcanization accelerator that does not function as a vulcanizing agent under a vulcanization condition (e.g., 150°C, 1.5 MPa) or at a temperature lower than 150°C and under a pressure lower than 1.5 MPa.

Examples of the sulfur non-releasing type sulfur atom-containing vulcanization accelerator include thiazole-based vulcanization accelerators not having $-S_n-$ ($n \geq 2$) (2-mercaptobenzothiazole (MBT), the zinc salt of 2-mercaptobenzothiazole (ZnMBT), the cyclohexylamine salt of 2-mercaptobenzothiazole (CMBT), etc.), sulfenamide-based vulcanization accelerators not having $-S_n-$ ($n \geq 2$) (N-cyclohexyl-2-benzothiazolylsulfenamide (CBS), N-(tert-butyl)-2-benzothiazolesulfenamide (TBBS), N,N-dicyclohexyl-2-benzothiazolylsulfenamide, etc.), tetramethylthiuram monosulfide (TMTM), and dithiocarbamate-based vulcanization accelerators not having $-S_n-$ ($n \geq 2$) (piperidinium pentamethylen-edithiocarbamate (PPDC), zinc dimethyldithiocarbamate (ZnMDC), zinc diethyldithiocarbamate (ZnEDC), zinc dibutyldithiocarbamate (ZnBDC), zinc N-ethyl-N-phenyldithiocarbamate (ZnEPDC), zinc N-pentamethylenedithiocarbamate (ZnPDC), sodium dibutyldithiocarbamate (NaBDC), copper dimethyldithiocarbamate (CuMDC), ferric dimethyldithiocarbamate (FeMDC), tellurium diethyldithiocarbamate (TeEDC), etc.). One of them may be used singly, or two or more of them may be used in combination. Among them, sulfenamide-based vulcanization accelerators not having $-S_n-$ ($n \geq 2$) are preferable, and N-(tert-butyl)-2-benzothiazolesulfenamide (TBBS) is more preferable. Di-2-benzothiazolyldisulfide (MBTS), which is a thiazole-based vulcanization accelerator, is a vulcanization accelerator that has $-S_n-$ ($n \geq 2$) and releases sulfur, but can be used as an equivalent to the sulfur non-releasing type sulfur atom-containing vulcanization accelerator, since di-2-benzothiazolyldisulfide (MBTS) does not function as a vulcanizing agent with respect to natural rubber or butadiene rubber when being blended in an ordinary amount.

In the rubber composition of the present invention, the amount of the sulfur atom-containing vulcanization accelerator to be added before the rubber component and the filler are kneaded is preferably not less than 1.0 part by mass and more preferably not less than 1.5 parts by mass, with respect to 100 parts by mass of the rubber component (the total amount of the rubber component to be used in all the steps), for the reason that a vulcanization reaction in a vulcanization step efficiently progresses. In addition, from the standpoint of inhibiting scorching or deposition on a surface, the amount is preferably not greater than 5 parts by mass and more preferably not greater than 3 parts by mass.

In the above production method, an additional sulfur donor may further be kneaded in a step other than the step before the rubber component and the filler are kneaded. By adding the additional sulfur donor, a crosslinking reaction can be caused to sufficiently progress during vulcanization while excessive progress of a crosslinking reaction during kneading is inhibited.

The additional sulfur donor is added, for example, at the latter stage of the step X or in the step Y in which the filler is added to the rubber component and kneading is performed at a kneading temperature of not lower than 120°C, or in the step F after the filler is added to the rubber component and kneading is performed at a kneading temperature of not lower than 120°C. The additional sulfur donor may be the same type as or different from the sulfur donor kneaded before the filler is added to the rubber component, and examples of the additional sulfur donor include powdery sulfur, precipitated sulfur, colloidal sulfur, surface-treated sulfur, and insoluble sulfur.

In the rubber composition of the present invention, the amount of the additional sulfur donor is not particularly limited. However, for the reason that a vulcanization reaction efficiently progresses in the vulcanization step, the amount of the additional sulfur donor with respect to 100 parts by mass of the rubber component (the total amount of the rubber component to be used in all the steps) is preferably not less than 0.5 parts by mass and more preferably not less than 0.8 parts by mass. For the reason that the wear resistance is excellent, the amount of the additional sulfur donor is preferably not greater than 3.0 parts by mass and more preferably not greater than 2.5 parts by mass.

In adding the additional sulfur donor, an additional vulcanization accelerator may be added. Examples of the additional vulcanization accelerator include: thiuram-based disulfides, polysulfides, and the like, which are sulfur atom-containing vulcanization accelerators; and guanidine-based, aldehyde-amine-based, aldehyde-ammonia-based, imidazoline-based vulcanization accelerators, which are vulcanization accelerators not having any sulfur atom.

In the rubber composition of the present invention, the amount of the additional vulcanization accelerator is not particularly limited. However, the amount of the additional vulcanization accelerator with respect to 100 parts by mass of the rubber component (the total amount of the rubber component to be used in all the steps) is preferably not less than 0.1 parts by mass and more preferably not less than 1 part by mass. In addition, the amount is preferably not greater than 5 parts by mass and more preferably not greater than 3 parts by mass.

The rubber composition (vulcanized rubber composition) of the present invention is obtained by vulcanizing an unvulcanized rubber composition obtained through the step F, by an ordinary method.

The rubber composition of the present invention can suitably be used for a tire member such as a tread, since the rubber composition has excellent wear resistance. In the case of producing a pneumatic tire by using the rubber composition of the present invention, the rubber composition of the present invention may be extruded in an unvulcanized state into the shape of a tread or the like, shaped on a tire forming machine by an ordinary method, and attached together with other tire members to form an unvulcanized tire (green tire), and then the unvulcanized tire may be heated and pressurized in a vulcanizer.

EXAMPLES

The present invention will be described in detail by means of examples, but the present invention is not limited to the examples.

Various chemicals used in synthesis of a monomer (1) and SBR (1) are listed below.

Cyclohexane: manufactured by Kanto Chemical Co., Inc.
Pyrrolidine: manufactured by Kanto Chemical Co., Inc.
Divinylbenzene: manufactured by Sigma-Aldrich Co. LLC.
1.6 M solution of n-butyllithium in hexane: manufactured by Kanto Chemical Co., Inc.
Isopropanol: manufactured by Kanto Chemical Co., Inc.
Styrene: manufactured by Kanto Chemical Co., Inc.
Butadiene: manufactured by Takachiho Chemical Industrial Co., Ltd.
Tetramethylethylenediamine: manufactured by Kanto Chemical Co., Inc.
Modifier: 3-(N,N-dimethylaminopropyl)trimethoxysilane manufactured by AZmax. Co.
2,6-tert-butyl-p-cresol: manufactured by Ouchi Shinko Chemical Industrial Co., Ltd.
Methanol: manufactured by Kanto Chemical Co., Inc.

(Synthesis of Monomer (1))

50 ml of cyclohexane, 4.1 ml of pyrrolidine, and 8.9 ml of divinylbenzene were added into a sufficiently nitrogen-purged 100-ml vessel. Then, 0.7 ml of the 1.6 M solution of n-butyllithium in hexane was added into the vessel at 0°C, and the mixture was stirred. After 1 hour, isopropanol was added thereinto to terminate the reaction, and extraction and purification were performed to obtain a monomer (1).

(Synthesis of SBR (1))

600 ml of cyclohexane, 12.6 ml of styrene, 71.0 ml of butadiene, 0.06 g of the monomer (1), and 0.11 ml of tetramethylethylenediamine were added into a sufficiently nitrogen-purged 1000-ml pressure-resistant vessel. Then, 0.2 ml of the 1.6 M solution of n-butyllithium in hexane was added into the vessel at 40°C, and the mixture was stirred. After 3 hours, 0.5 ml of the modifier was added into the vessel, and the mixture was stirred. After 1 hour, 3 ml of isopropanol was added thereinto to terminate the polymerization. After 1 g of 2,6-tert-butyl-p-cresol was added to the reaction solution, reprecipitation was performed with methanol, and the resultant precipitate was dried by heating to obtain SBR (1).

Various chemicals used in Example and Comparative Examples are listed below.

SBR(1): synthesized by the above method (Mw: 300 thousand)
SBR(2): SLR6430 (Mw: 1.2 million) manufactured by the Dow Chemical Company
BR: BR150B (Mw: 400 thousand) manufactured by Ube Industries, Ltd.
Vulcanization accelerator: Nocceler NS (N-(tert-butyl)-2-benzothiazolesulfenamide (TBBS) manufactured by Ouchi Shinko Chemical Industrial Co., Ltd.
Sulfur donor: Nocceler TRA (dipentamethylenethiuram tetrasulfide (DPTT)) manufactured by Ouchi Shinko Chemical Industrial Co., Ltd.
Silica: ULTRASIL VN3 manufactured by Evonik Industries Ag
Silane coupling agent: Si266 manufactured by Evonik Industries Ag
Carbon black: DIABLACK I manufactured by Mitsubishi Chemical Corporation
Oil: Diana Process AH-24 manufactured by Idemitsu Kosan Co., Ltd.
Zinc oxide: zinc oxide manufactured by Mitsui Mining & Smelting Co., Ltd.
Stearic acid: stearic acid "Tsubaki" manufactured by NOF Corporation
Antioxidant: OZONONE 6C (N-(1,3-dimethylbutyl)-N'-phenyl-p-phenylenediamine) manufactured by Seiko Chemical Co., Ltd.
Sulfur: powdery sulfur manufactured by Tsurumi Chemical Industry Co., Ltd.

(Example and Comparative Examples)

According to the composition shown in Table 1, various chemicals shown in Step X were kneaded in a 1.7-L Banbury mixer at a discharge temperature of 100°C for 5 minutes (step X). Next, the kneaded product in the step X and various chemicals shown in Step Y were kneaded in the 1.7-L Banbury mixer at a temperature of not lower than 140°C for 30 seconds, and were further kneaded at a discharge temperature of 150°C for 3 minutes (step Y). Then, the kneaded product in the step Y and various chemicals shown in Step F were kneaded at approximately 80°C for 3 minutes by using an open roll (step F) to obtain an unvulcanized rubber composition. The obtained unvulcanized rubber composition was extruded into the shape of a tread by using an extruder having a die with a predetermined shape, and attached together with other tire members to form an unvulcanized tire, and press vulcanization was performed under a condition of 170°C for 12 minutes, to produce a test tire (tire size: 195/65R15). The obtained test tire was evaluated as described below. The results are shown in Table 1.

1. SANS Measurement Method

A plate-like sample (molded product) having a thickness of about 1 mm was swollen to equilibrium in deuterated toluene, and mounted to a sample holder. The sample was then irradiated with neutron rays at room temperature. Absolute scattering intensity curves obtained by measuring the sample at distances of 2.5 m and 10 m from the sample to a detector and by measuring the sample with a focusing lens were combined by the least squares method. These three curves were combined by fixing the scattering intensity curve obtained by measuring the sample at a distance of 2.5 m from the sample to the detector, and shifting the scattering intensity curves obtained by measuring the sample at a distance 10 m from the sample to the detector and by measuring the sample with the focusing lens. Curve-fitting was performed on the obtained scattering intensity curve I(q) by using (Expression 1-2) to (Expression 1-7), a fitting parameter $\Xi_i$ (a correlation length of 10 nm to 100 $\mu$m (the size of the ununiform network structure of the polymer)) was obtained by a least-squares method, and a maximum value $\Xi_c$ of the correlation length $\Xi_i$ and a standard deviation $\sigma_c$ thereof were calculated.

(SANS device)

SANS: the SANS measurement device attached to the beamline SANS-J at JRR-3 of the Japan Atomic Energy Agency, which is an independent administrative agency.

(Measurement Conditions)

Wavelength of neutron rays: 6.5 Å
Neutron flux intensity of neutron rays: $9.9 \times 10^7$ neutrons/cm$^2$/s
Distance from sample to detector: 2.5 m and 10 m (further, in order to obtain information at smaller angles, measurement was performed at a distance of 10 m from the sample to the detector, using the focusing lens)

(Detector)

Two-dimensional detector ($^3$He two-dimensional detector and two-dimensional photomultiplier + ZnS/$^6$LiF detector)

2. Weight Average Molecular Weight of Rubber Component

The weight average molecular weight of a mixture of the rubber components used in each example was obtained by conversion, using polystyrene standard, of a value measured by gel permeation chromatography (GPC) (GPC-8000 series manufactured by Tosoh Corporation, detector: differential refractometer, column: TSKgel SuperMultipore HZ-M manufactured by Tosoh Corporation).

3. Wear Resistance on Actual Vehicle

The above test tire was mounted to a front wheel drive vehicle produced in Japan. The groove depth of the tire tread portion was measured after running for 8000 km, and a running distance when the tire groove depth was reduced by 1 mm was calculated, and indexed by the following expression. The higher the index is, the more favorable the wear resistance is.

(Running distance when tire groove of each composition was reduced by 1 mm) /

(Running distance when tire groove of Comparative Example 1 was reduced by 1 mm) × 100

[Table 1]

| | | Example | Comparative Example | | |
|---|---|---|---|---|---|
| | | 1 | 1 | 2 | 3 |
| Composition (part(s) by mass) | Step X | | | | |
| | SBR (1) | 20 | 80 | 80 | 20 |
| | SBR (2) | 60 | - | - | 60 |
| | BR | 20 | 20 | 20 | 20 |
| | Vulcanization accelerator | 1 | - | 1 | - |
| | Sulfur donor | 1.5 | - | 1.5 | - |
| | Step Y | | | | |
| | Silica | 70 | 70 | 70 | 70 |
| | Silane coupling agent | 6 | 6 | 6 | 6 |
| | Carbon black | 5 | 5 | 5 | 5 |
| | Oil | 15 | 15 | 15 | 15 |
| | Zinc oxide | 5 | 5 | 5 | 5 |
| | Stearic acid | 3 | 3 | 3 | 3 |
| | Antioxidant | 2 | 2 | 2 | 2 |
| | Vulcanization accelerator | 1 | 1 | 1 | 1 |
| | Sulfur donor | 1.5 | 1.5 | 1.5 | 1.5 |
| | Step F | | | | |
| | Sulfur | 1.5 | 1.5 | 1.5 | 1.5 |
| | Vulcanization accelerator | - | 1 | - | 1 |
| | Sulfur donor | - | 1.5 | - | 1.5 |
| Evaluation | $\sigma_c$ (nm) | 135 | 201 | 169 | 496 |
| | $\Xi_c$ (nm) | 945 | 921 | 916 | 925 |
| | Weight average molecular weight of rubber component ($10^4$) | 150 | 30 | 30 | 150 |
| | Wear resistance on actual vehicle (index) | 155 | 100 | 121 | 115 |

From Table 1, it is found that $\sigma_c$ and the wear resistance have a high correlation, and the wear resistance becomes more favorable as $\sigma_c$ decreases. In addition, it is found that particularly favorable wear resistance is achieved in the Example which satisfies a relationship of $\sigma_c \leq \Xi_c/2$ and contains a rubber component having a weight average molecular weight of not less than one million.

## Claims

1. A rubber composition in which a relationship between: a maximum value $\Xi_c$ of a correlation length $\Xi_i$ of 10 nm to 100 μm obtained by performing curve-fitting with the following (Expression 1-2) to (Expression 1-7) on a scattering intensity curve I(q) obtained by performing X-ray scattering measurement or neutron scattering measurement in a region of q represented by the following (Expression 1); and a standard deviation $\sigma_c$ of the maximum value $\Xi_c$ is $\sigma_c \leq \Xi_c/2$, the rubber composition containing a rubber component having a weight average molecular weight of not less than one million, and wherein an amount of carbon black with respect to 100 parts by mass of the rubber component is not less than 1 part by mass and is not greater than 20 parts by mass,
[Math. 1]

(Expression 1)

$$q = \frac{4\pi \sin(\theta/2)}{\lambda}$$

13

($\theta$: scattering angle, $\lambda$: a wavelength of X-rays or neutron rays)

[Math. 2]

(Expression 1-2)

$$I_{(q)} = \int \frac{A}{1 + q^2 \xi^2} g_{a(r)} dr + \sum_{i=1}^{n} \int \frac{B_i}{(1 + q^2 \Xi_i^2)^2} g_{bi(r)} dr$$

(Expression 1-3)

$$\xi < \Xi_i$$

(Expression 1-4)

$$A = 8\pi N_a \rho^2 \xi^3$$

(Expression 1-5)

$$B_i = 4\pi N_i \rho^2 \Xi_i^2$$

(Expression 1-6)

$$g_{a(r)} = \frac{1}{\sqrt{2\pi\sigma_a^2}} exp\left(-\frac{(r-\xi)^2}{2\sigma_a^2}\right)$$

(Expression 1-7)

$$g_{bi(r)} = \frac{1}{\sqrt{2\pi\sigma_i^2}} exp\left(-\frac{(r-\Xi_i)^2}{2\sigma_i^2}\right)$$

(A, $B_i$, $\xi$, $\Xi_i$: fitting parameters)

(n: an integer)

(q: the same as in (Expression 1))

($N_a$: the number of scatterers having a correlation length $\xi$ per unit volume (number/cm$^3$))

($N_i$: the number of scatterers having the correlation length $\Xi_i$ per unit volume (number/cm$^3$))

(p: a scattering length density difference (cm$^{-2}$) or an electron density difference (electron cm$^{-3}$) between the scatterers and a deuterated solvent or air therearound)

(ga(r): a probability density function having an average correlation length $\xi$ and a standard deviation $\sigma_a$, when a correlation length is assumed to be present in a Gaussian distribution)

(gbi(r): a probability density function having an average correlation length $\Xi_i$ and a standard deviation $\sigma_i$ when a correlation length is assumed to be present in a Gaussian distribution).

2. The rubber composition according to claim 1, wherein the rubber component is a polymer of one or more conjugated diene compounds.

3. The rubber composition according to claim 1 or 2, wherein the rubber composition is used for a tire member.

4. The rubber composition according to any one of claims 1 to 3, wherein the X-ray scattering measurement is small-angle X-ray scattering measurement, and the neutron scattering measurement is small-angle neutron scattering measurement.

5. The rubber composition according to any one of claims 1 to 4, wherein the X-ray scattering measurement or the neutron scattering measurement is performed in a region of q that is not greater than 10 nm$^{-1}$.

6. A production method for the rubber composition according to any one of claims 1 to 5, the production method comprising the step of kneading the rubber component, a sulfur donor, and a sulfur atom-containing vulcanization accelerator before the rubber component and a filler are kneaded, then adding the filler to an obtained kneaded product, and performing kneading at a kneading temperature of not lower than 120°C.

**Patentansprüche**

1. Kautschukzusammensetzung, in welcher zwischen: einem Maximalwert $\Xi_c$ einer Korrelationslänge $\Xi_i$ von 10 nm bis 100 $\mu$m, erhalten durch das Fitten einer Kurve mittels (Ausdruck 1-2) bis (Ausdruck 1-7) an eine Streuintensitätskurve I(q), erhalten durch Röntgenstreuungsmessung oder Neutronenstreuungsmessung in einem Bereich von q, dargestellt durch den folgenden (Ausdruck 1); und einer Standardabweichung $\sigma_c$ des Maximalwertes $\Xi_c$ ein Verhältnis $\sigma_c \leq \Xi_c / 2$ besteht, wobei die Kautschukzusammensetzung eine Kautschukkomponente mit einem gewichtsmittleren Molekulargewicht von nicht weniger als einer Million enthält, und wobei eine Menge an Ruß, bezogen auf 100 Massenteile der Kautschukkomponente, nicht weniger als 1 Massenteil und nicht mehr als 20 Massenteile beträgt,
[Math. 1]

(Ausdruck 1)

$$q = \frac{4\pi \sin(\theta/2)}{\lambda}$$

($\theta$: Streuwinkel, $\lambda$: eine Wellenlänge der Röntgenstrahlen oder Neutronenstrahlen)
[Math. 2]

(Ausdruck 1-2)

$$I_{(q)} = \int \frac{A}{1 + q^2\xi^2}\, g_{a(r)}dr + \sum_{i=1}^{n} \int \frac{B_i}{(1 + q^2\Xi_i^2)^2}\, g_{bi(r)}dr$$

(Ausdruck 1-3)

$$\xi < \Xi_i$$

(Ausdruck 1-4)

$$A = 8\pi N_a \rho^2 \xi^3$$

(Ausdruck 1-5)

$$B_i = 4\pi N_i \rho^2 \Xi_i^2$$

(Ausdruck 1-6)

$$g_{a(r)} = \frac{1}{\sqrt{2\pi\sigma_a^2}} exp\left(-\frac{(r-\xi)^2}{2\sigma_a^2}\right)$$

(Ausdruck 1-7)

$$g_{bi(r)} = \frac{1}{\sqrt{2\pi\sigma_i^2}} exp\left(-\frac{(r-\Xi_i)^2}{2\sigma_i^2}\right)$$

(A, Bi, $\xi$, $\Xi_i$: Fittingparameter)
(n: eine ganze Zahl)
(q: wie in (Ausdruck 1))
($N_a$: Anzahl der Streuer mit einer Korrelationslänge $\xi$ pro Volumeneinheit (Anzahl/cm$^3$))
($N_i$: Anzahl der Streuer mit der Korrelationslänge $\Xi_i$ pro Volumeneinheit (Anzahl/cm$^3$))
(p: eine Differenz einer Streuungslängendichte (cm$^{-2}$) oder einer Elektronendichte (Elektronen cm$^{-3}$) zwischen den Streuern und einem deuterierten Lösungsmittel oder Luft in der Umgebung)
(ga(r): eine Wahrscheinlichkeitsdichtefunktion mit einer mittleren Korrelationslänge $\xi$ und einer Standardabweichung $\sigma_a$ unter der Annahme, dass eine Korrelationslänge in einer Gauss-Verteilung vorliegt)
(gbi(r): eine Wahrscheinlichkeitsdichtefunktion mit einer mittleren Korrelationslänge $\Xi_i$ und einer Standardabweichung $\sigma_i$ unter der Annahme, dass eine Korrelationslänge in einer Gauss-Verteilung vorliegt).

2. Kautschukzusammensetzung nach Anspruch 1, wobei die Kautschukkomponente ein Polymer einer oder mehrerer konjugierter Dienverbindungen ist.

3. Kautschukzusammensetzung nach Anspruch 1 oder 2, wobei die Kautschukzusammensetzung für ein Reifenteil verwendet wird.

4. Kautschukzusammensetzung nach einem der Ansprüche 1 bis 3, wobei die Röntgenstreuungsmessung Kleinwinkelröntgenstreuungsmessung und die Neutronenstreuungsmessung Kleinwinkelneutronenstreuungsmessung ist.

5. Kautschukzusammensetzung nach einem der Ansprüche 1 bis 4, wobei die Röntgenstreuungsmessung oder die Neutronenstreuungsmessung in einem Bereich von q durchgeführt wird, der nicht größer ist als 10 nm$^{-1}$.

6. Herstellungsverfahren für die Kautschukzusammensetzung nach einem der Ansprüche 1 bis 5, wobei das Herstellungsverfahren umfasst: den Schritt des Knetens der Kautschukkomponente, eines Schwefeldonors und eines Schwefelatom-haltigen Vulkanisationsbeschleunigers, bevor die Kautschukkomponente und ein Füllstoff verknetet werden, dann das Hinzufügen des Füllstoffes zum erhaltenen gekneteten Produkt, und das Durchführen eines Knetvorgangs bei einer Knettemperatur von nicht weniger als 120 °C.

**Revendications**

1. Composition de caoutchouc dans laquelle une relation entre : une valeur maximale $\Xi_c$ d'une longueur de corrélation $\Xi_i$ de 10 nm à 100 $\mu$m obtenue en effectuant un ajustement de courbe avec les (Expression 1-2) à (Expression 1-7) suivantes sur une courbe d'intensité de diffusion I(q) obtenue en effectuant une mesure de diffusion des rayons X ou de diffusion des neutrons dans une région q représentée par l'(Expression 1) suivante ; et un écart-type $\sigma_c$ de la valeur maximale $\Xi_c$ est $\sigma_c < \Xi_c/2$, la composition de caoutchouc contenant un composant caoutchouc ayant une masse moléculaire moyenne en poids non inférieure à un million, et dans laquelle une quantité de noir de carbone par rapport à 100 parties en masse du composant caoutchouc n'est pas inférieure à 1 partie en masse et n'est pas supérieure à 20 parties en masse,

[Math. 1]

(Expression 1)

$$q = \frac{4\pi \sin(\theta/2)}{\lambda}$$

($\theta$ : angle de diffusion, $\lambda$ : longueur d'onde des rayons X ou des rayons neutroniques)

[Math. 2]

(Expression 1-2)

$$I_{(q)} = \int \frac{A}{1 + q^2\xi^2} g_{a(r)}dr + \sum_{i=1}^{n} \int \frac{B_i}{(1 + q^2\Xi_i^2)^2} g_{bi(r)} dr$$

(Expression 1-3)

$$\xi < \Xi_i$$

(Expression 1-4)

$$A = 8\pi N_a \rho^2 \xi^3$$

(Expression 1-5)

$$B_i = 4\pi N_i \rho^2 \Xi_i^2$$

(Expression 1-6)

$$g_{a(r)} = \frac{1}{\sqrt{2\pi\sigma_a^2}} exp\left(-\frac{(r-\xi)^2}{2\sigma_a^2}\right)$$

(Expression 1-7)

$$g_{bi(r)} = \frac{1}{\sqrt{2\pi\sigma_i^2}} exp\left(-\frac{(r-\Xi_i)^2}{2\sigma_i^2}\right)$$

(A, $B_i$, $\xi$, $\Xi_i$ : paramètres d'ajustement)
(n : un entier)
(q : le même que dans (Expression 1))
($N_a$ : nombre de diffuseurs ayant une longueur de corrélation $\xi$ par unité de volume (nombre/cm$^3$))
($N_i$ : nombre de diffuseurs ayant la longueur de corrélation $\Xi_i$ par unité de volume (nombre/cm$^3$))
(p : une différence de densité de longueur de diffusion (cm$^{-2}$) ou une différence de densité d'électrons (électron cm$^{-3}$) entre les diffuseurs et un solvant deutéré ou l'air autour de ceux-ci)
(ga(r) : une fonction de densité de probabilité ayant une longueur de corrélation moyenne $\xi$ et un écart-type $\sigma_c$ lorsqu'une longueur de corrélation est supposée être présente dans une distribution gaussienne)

(gbi(r) : une fonction de densité de probabilité ayant une longueur de corrélation moyenne $\xi$ et un écart-type $\sigma_i$ lorsqu'une longueur de corrélation est supposée être présente dans une distribution gaussienne).

2. Composition de caoutchouc selon la revendication 1, dans laquelle le composant caoutchouc est un polymère d'un ou plusieurs composés diéniques conjugués.

3. Composition de caoutchouc selon la revendication 1 ou 2, laquelle composition de caoutchouc est utilisée pour un élément de pneumatique.

4. Composition de caoutchouc selon l'une quelconque des revendications 1 à 3, dans laquelle la mesure de diffusion des rayons X est une mesure de diffusion des rayons X aux petits angles et la mesure de diffusion des neutrons est une mesure de diffusion des neutrons aux petits angles.

5. Composition de caoutchouc selon l'une quelconque des revendications 1 à 4, dans laquelle la mesure de diffusion des rayons X ou la mesure de diffusion des neutrons est effectuée dans une région q qui n'est pas plus grande que 10 nm$^{-1}$.

6. Procédé de production de la composition de caoutchouc selon l'une quelconque des revendications 1 à 5, le procédé de production comprenant l'étape consistant à malaxer le composant caoutchouc, un donneur de soufre et un accélérateur de vulcanisation contenant des atomes de soufre avant que le composant caoutchouc et une charge soient malaxés, puis à ajouter la charge à un produit malaxé obtenu et à effectuer un malaxage à une température de malaxage non inférieure à 120 °C.

Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013032497 A
- EP 2735865 A
- EP 2749874 A
- EP 2803982 A
- EP 1958971 A
- EP 2098554 A
- EP 2557410 A
- EP 2799482 A